# EUROPÄISCHE PATENTSCHRIFT

(11) **EP 2 440 259 B1**
(45) Veröffentlichungstag und Bekanntmachung des Hinweises auf die Patenterteilung: **27.03.2013**
(21) Anmeldenummer: 10700966.4
(22) Anmeldetag: 06.01.2010
(51) Int. Cl.: A61L 9/12, E03D 9/00, A47K 10/32, A47K 10/22

(54) **DUFTABGABESYSTEM FÜR EINE PAPIERROLLE**
FRAGRANCE DISPENSER SYSTEM FOR A PAPER ROLL
SYSTÈME D'APPLICATION DE PARFUM SUR UN ROULEAU DE PAPIER

(30) Priorität: 09.06.2009 DE 102009026859
(43) Veröffentlichungstag der Anmeldung: 18.04.2012
(73) Patentinhaber: Henkel AG & Co. KGaA, 40589 Düsseldorf (DE)
(72) Erfinder: MEIER, Frank, 40589 Düsseldorf (DE)
(86) Internationale Anmeldenummer: PCT/EP2010/050067
(87) Internationale Veröffentlichungsnummer: WO 2010/142469

(56) Entgegenhaltungen:
- US-A- 5 170 938
- US-A- 5 422 078
- US-A- 5 857 621
- US-A1- 2005 224 594

## Beschreibung

Die vorliegende Erfindung betrifft ein Duftabgabesystem zur Desodorierung und/oder zur Beduftung von Räumen nach dem Oberbegriff von Anspruch 1. Derartige Duftabgabesysteme werden an drehbar gelagerten Papierrollen, insbesondere an Toilettenpapierrollen oder an Haushaltspapierrollen angewendet und umfassen einen mit einer Mehrzahl von Öffnungen versehenen Behälter, der einen widerverschließbaren, ringförmigen Aufnahmeraum umschließt, in den eine mit einer Duftstoff-permeablen Membran verschlossene und mit einer fließfähigen, wenigstens einen Duftstoff enthaltenden Zubereitung befüllte ringförmige Blisterverpackung positionierbar ist.

In Toilettenräumen treten insbesondere bei fehlender Frischluftzufuhr häufig unangenehme Gerüche auf. Um die Toilettenluft frisch zu halten und die störenden Gerüche zu beseitigen oder möglichst weitgehend zu verringern sind neben Duftsprays verschiedenartige Raumbedufter bekannt.

Diese Raumbedufter können beispielsweise ein mit Parfüm oder einer sonstigen duftabgebenden Formulierung imprägnierter Festkörper sein, der üblicherweise zu dekorativen Zwecken eine bestimmte Formgebung bzw. optische Gestaltung aufweist. Ein derartiger Raumbedufter, der unmittelbar an einer Toilettenpapierrolle eingesetzt wird, ist aus der US 5 170 938 bekannt. Er umfasst zwei an die beiden Stirnseiten einer Toilettenpapierrolle aufsteckbare Scheiben, aus denen zu Dekorationszwecken jeweils verschiedene Muster, Symbole oder Figuren ausgestanzt sind. Diese Scheiben, die auch einzeln einsetzbar sind, können entweder direkt mit einer Parfüm-Formulierung imprägniert sein oder sie können als Auflage für einen separaten Duftträger dienen, der seinerseits mit einer Parfüm-Formulierung imprägniert ist. Ein derartiger Raumbedufter weist jedoch über seine Wirkzeit eine nachlassende Duftintensität und somit eine abnehmende Beduftung der Umgebungsluft auf. Darin liegt ein übliches Problem der gängigen passiven Duftspender, die alleine eine diffusionsbedingte Nachlieferung des Duftes aus dem dufttragenden Material ermöglichen.

Andere Raumbedufter umfassen ein mit Öffnungen versehenes Gehäuse, das mit verschiedenen Luft reinigenden und/oder Duft abgebenden Substanzen gefüllt sein kann.

So schlägt die WO 2004/020004 ein Duftabgabesystem vor, welches in einem rotationssymmetrischen Behälter mit mondsichelartiger Querschnittsform eine Vielzahl von Duftstoff-tragenden Partikeln enthält. Das System kann beispielsweise über eine angebrachte Haltevorrichtung an einer geeigneten Stelle in einer Toilette aufgehängt werden und dort Duft abgeben.

Aus der US 2 753 209 ist ein Haltekern für eine Toilettenpapierrolle bekannt, der ein Gehäuse für einen einliegenden zylindrischen Duftträgerstift bildet. Der Haltekern besteht aus zwei über Finger axial ineinander greifenden Kernhälften, die mittels einer Schraubenfeder auseinander gedrückt werden, um jeweils mit einer außenseitig axial vorspringenden Nabe in eine Aufnahmebohrung einer Halterung für Toilettenpapierrollen einzugreifen.

Zwischen den beiden Kernhälften sind dabei mehrere axiale Öffnungen vorgesehen, durch die die Duftstoffe des Duftträgerstifts aus dem Haltekern austreten können.

Von Nachteil an dieser Ausführungsform ist die relativ aufwendige Konstruktion, wodurch der Raumbedufter nicht nur teuer in der Herstellung, sondern aufgrund der Reibung zwischen den bewegten Kernhälften auch Verschleißerscheinungen unterworfen und somit anfällig für Funktionsstörungen ist. Außerdem weist auch dieser Raumbedufter als passiven Duftspender über seine Wirkzeit eine kontinuierlich nachlassende Duftintensität und damit eine abnehmende Beduftung der Umgebungsluft auf.

Es wäre jedoch wünschenswert, wenn jeweils eine kurzzeitige Erhöhung der Duftabgabe erreicht werden könnte, wenn die Umgebungsluft besonders stark mit unangenehmen Gerüchen belastet ist.

Dazu schlägt die US 6 688 551 ein Duftabgabesystem vor, bei dem der Haltekern für Toilettenpapierrollen ein internes Reservoir für eine hochintensive Parfüm-Formulierung aufweist, die über ein internes Transportsystem zu einer oder mehreren Austrittsöffnungen geleitet wird. Auch dieser Haltekern umfasst zwei Kernhälften mit stirnseitigen Naben, die über eine Feder axial auseinander gedrückt werden, so dass der Haltekern nahezu drehfest in einer Halterung für Toilettenpapierrollen fixierbar ist. Beim Abrollen von Toilettenpapier wird aufgrund von Luftturbulenzen eine erhöhte Menge der Parfüm-Formulierung abgegeben, damit es zu einer kurzzeitig verstärkten Duftabgabe kommt.

Auch dieses Duftabgabesystem weist aufgrund des Federmechanismus und des internen Transportsystems eine relativ aufwendige Konstruktion auf, was sich in höheren Produktionskosten nachteilig niederschlägt. Außerdem fällt die beabsichtigte kurzzeitige Erhöhung nicht immer in dem gewünschten Maße aus.

Ein weiteres Duftabgabesystem mit einer bei Bedarf erhöhten Duftabgabe ist aus der US 4 497 456 bekannt. Dieses Duftabgabesystem der eingangs genannten Art umfasst ebenfalls einen Haltekern für eine Toilettenpapierrolle, der einen mit mehreren Öffnungen versehenen Behälter zur Aufnahme einer Vielzahl von kleinen Duftträgerpartikeln bildet. Dieser Haltekern ist frei drehbar in der Toilettenpapierhalterung gelagert, so dass das Abrollen von Toilettenpapier aufgrund von Reibungserscheinungen auch Drehbewegungen des Haltekerns hervorruft. Während der Drehung des Haltekerns fallen die Duftträgerpartikel durcheinander, was zu neuen Anordnungen der einzelnen Duftträgerpartikel relativ zueinander führt. Dadurch werden stets neue Oberflächenbereiche der Duftträgerpartikel freigelegt, was als sogenannter Booster-Effekt in der gewünschten kurzzeitig verstärkten Duftabgabe resultiert.

Da der Haltekern sich jedoch frei, also unabhängig von der Toilettenpapierrolle drehen kann, ist eine zuverlässige Wirkung des Booster-Effekts nicht immer gewährleistet. Insbesondere wenn Papier mit einer höheren Geschwindigkeit abgezogen wird, könnte die gewünschte Drehbewegung des Haltekerns nur in einem deutlich geringeren Maße ausfallen oder sogar vollständig unterbleiben.

Ein weiteres, ähnlich konstruiertes System ist aus der US 4 759 510 bekannt Dieses Patent beschreibt einen Haltekern für Toilettenrollen, der als Behälter für Duftträgerpartikel ausgebildet ist, welche beim Drehen des Kerns durcheinanderfallen und Duft abgeben.

Ein generelles Problem bei derartigen Raumbeduftern in Form von Haltekernen liegt ferner darin, dass sie nur bei solchen Halterungen von Papierrollen einsetzbar sind, bei denen der ursprüngliche Haltekern vollständig entfernbar ist. Modernen Halterungen haben jedoch oftmals eine einseitig befestigte, insbesondere einstückig mit der Halterung ausgeführte und vorzugsweise relativ dünn ausgebildete Halteachse, auf die eine Papierrolle axial aufgeschoben wird. Bei derartigen modernen Halterungen sind die vorgenannten Duftabgabesysteme mit auszuwechseindem Haltekern daher nicht einsetzbar.

Ein weiterer Nachteil der Duftabgabesysteme mit auswechselbarem Haltekern besteht darin, dass die Duftstoffe nur innerhalb des Kernrohrs der Papierrolle austreten und daher die Umgebungsluft nur über Umwege und in beschränktem Maße erreichen können. Die Desodorierung bzw. die Beduftung des mit unangenehmen Gerüchen belasteten Raumes kann daher eine optimale Wirksamkeit nicht erreichen.

Einen an einer Stirnseite einer Toilettenpapierrolle anbringbaren Lufterfrischer mit einem festen Duftstoffträger offenbart US2005/0224594A1 und US5170938. US5857621 beschreibt ein Bevorratungs- und Beduftungssystem für Toilettenpapierrollen.

Aufgabe der vorliegenden Erfindung ist es daher, ein konstruktiv einfaches, preiswert herzustellendes und leicht handhabbares und nachfüllbares Duftabgabesystem der eingangs genannten Art zu schaffen, das auch bei Papierrollen mit nicht auswechselbarem Haltekern einsetzbar ist und das bei einer stets effektiven und hochwirksamen Raumbeduftung zuverlässig eine kurzzeitig erhöhte Duftabgabe beim Abrollen von Papier ermöglicht

Diese Aufgabe wird erfindungsgemäß durch ein Duftabgabesystem nach Anspruch 1 gelöst. Vorteilhafte Ausgestaltungen und Weiterbildungen der Erfindung ergeben sich aus den abhängigen Ansprüchen.

Wesentlich bei der erfindungsgemäßen Lösung ist es, dass der Behälter ringförmig ausgebildet ist und einen ringförmigen Aufnahmeraum umschließt und dass der Behälter kraftschlüssig und/oder formschlüssig wirkende Befestigungsmittel zur drehfesten Verbindung des Behälters mit einer Papierrolle und/oder mit dem Kernrohr einer Papierrolle aufweist.

Der Hauptvorteil liegt dabei darin, dass ein ringförmig ausgebildeter Behälter zusammen mit einer Papierrolle auch auf feststehende Halteachsen von Papierrollenhalterungen aufschiebbar ist. So kann ein gemäß der vorliegenden Erfindung ringförmig ausgebildeter Behälter beispielsweise zusammen mit einer Toilettenpapierrolle auch auf eine feststehende Halteachse einer Toilettenpapierrollenhalterung aufgeschoben werden. Dennoch kann der Behälter auch auf den herausnehmbaren beidseitig gelagerten Haltekernen älterer Bauart verwendet werden. Deshalb kann das erfindungsgemäße Duftabgabesystem insbesondere bei allen bekannten Typen von Toilettenpapierrollenhalterungen eingesetzt werden.

Dabei kommt es nicht auf den Querschnitt des ringförmigen Aufnahmeraums an. So kann der Querschnitt beispielsweise oval, aber auch mit Geraden und/oder Ecken ausgebildet sein. Insbesondere kann der Aufnahmeraum in der Form eines Torus ausgeführt sein. Wesentlich ist lediglich, dass der Behälter in der Form eines Ringes mit einem für herkömmliche Achsen und Kernrollen ausreichend groß dimensionierten freien Innenraum ausgeführt ist, damit er ohne Klemmerscheinungen frei drehbar auf eine Achse bzw. Kernrolle einer Papierrollenhalterung aufgesteckt werden kann.

Ein weiterer wesentlicher Vorteil besteht darin, dass aufgrund der rotatorischen, Befestigung des ringförmigen Behälters an der Papierrolle unabhängig von der Abrollgeschwindigkeit stets eine ausreichende Drehbewegung des Behälters und somit zuverlässig die gewünschte Erhöhung der Raumbeduftung beim Abrollen von Papier erzielt wird.

Das erfindungsgemäße Duftabgabesystem zeichnet sich durch eine konstante Duftstoffabgabe in der Ruheposition der Papierrolle und einen besonders vorteilhaften, weil sofort wirkenden und nach der Drehung langsam abklingenden Duftboost beim Drehen der Papierrolle aus. Dies wird durch Benetzung der gesamten Membranoberfläche mit viskoser Zubereitung beim Drehen und ein durch die Viskosität bedingtes Anhaften der Zubereitung auf der Membranoberfläche bewirkt.

Hierzu ist die kinematische Viskosität der in der Blisterverpackung bevorrateten Zubereitung bei 40°C zwischen 1,4 und 150 mm2/s, bevorzugt 1,4 und 44 mm2/s eingestellt, wobei das Volumen der Blisterverpackung mit weniger als 95% der Zubereitung befüllt ist.

Die kinematische Viskosität wird gemäß DIN EN ISO 3104 bestimmt.

Durch die Befüllung der ringförmigen Blisterverpackung mit Duftstoff enthaltener Zubereitung von weniger als 95% ist ferner eine leichte olfaktometrische Unterscheidung zwischen der Duftstoffabgabe in Ruhestellung der Papierrolle und während bzw. nach der Drehung der Papierrolle ermöglicht.

Ferner kann die mit Duftstoff befüllte, ringförmige Blisterverpackung nach Gebrauch auf einfache Weise aus dem Duftabgabesystem entnommen und durch eine neue, frisch befüllte Blisterverpackung ausgetauscht werden.

Das erfindungsgemäße Duftabgabesystem ist bei einfacher Konstruktion kostengünstig herzustellen und leicht zu montieren bzw. einfach zu handhaben. Zur Anbringung an einer Papierrolle können die Befestigungsmittel vorzugsweise so gestaltet sein, dass ein einfaches axiales Aufstecken des ringförmigen Behälters auf eine Stirnseite einer Papierrolle die beabsichtigte drehfeste Verbindung mit der Papierrolle oder mit dem üblicherweise aus Pappe bestehenden Kernrohr einer Toilettenpapierrolle oder einer Küchenpapierrolle erzeugt. Zusätzlich kann hierdurch auch ein kraftschlüssiger und/oder formschlüssiger Halt in axialer Richtung sichergestellt werden.

Des Weiteren ist es von Vorteil, dass das Duftabgabesystem ein vergleichsweise geringes Gewicht aufweist, wodurch beim Drehen der Papierrolle nur eine vergleichsweise geringe Unwucht entsteht und die Papierrolle nicht aus ihrer vorgesehenen Führung gedrückt bzw. geschoben wird.

Für eine besonders leichte und vorzugsweise konzentrische Positionierung des ringförmigen Behälters an einer Papierrolle schlägt die vorliegende Erfindung weiterhin vor, dass der Behälter eine koaxial vorstehende Hülse aufweist, die von einer Stirnseite einer Papierrolle axial in die Papierrolle oder in das Kernrohr einer Papierrolle einsteckbar ist. Dabei kann die Hülse entweder als separates Bauteil an dem Behälter befestigt oder einstückig mit dem Behälter oder einem Teil des Behälters ausgebildet sein.

Besonders vorteilhaft ist es dabei ferner, wenn die Hülse konusförmig ausgebildet ist, so dass ihr freies Ende mit einem kleineren Außendurchmesser besonders leicht in das Kernrohr einer Papierrolle eingeführt werden kann.

Gleichzeitig kann auf diese Weise ein kraftschlüssiger Halt an Papierrollen mit unterschiedlichen Kernrohr-Innendurchmessern erreicht werden.

Die Anpassbarkeit an verschiedene Innendurchmesser von Papierrollen kann dabei weiter verbessert werden, wenn die Hülse zwei oder mehr zumindest im wesentlichen axiale Trennschlitze aufweist, die sich ausgehend von dem freien Ende der Hülse über mindestens einen Teil ihrer axialen Länge erstrecken, so dass der freie Endbereich der Hülse in zwei oder mehr Umfangsabschnitte unterteilt ist, die sich relativ leicht elastisch nach innen biegen lassen.

Eine besonders bevorzugte Art der Befestigungsmittel sieht vor, dass an der Hülse mindestens ein elastisch nach außen federndes Halteelement vorgesehen ist. Vorzugsweise erstrecken sich die Halteelemente als keilförmige Haltestege radial nach außen, die beim Einstecken in ein Kernrohr einer Papierrolle zunehmend nach innen gedrückt werden und so zu einer kraftschlüssigen Befestigung führen. Alternativ oder ergänzend hierzu können die Befestigungsmittel auch Haken und/oder Klammern umfassen.

Der Behälter kann grundsätzlich auf unterschiedliche Weise hergestellt werden, er kann beispielsweise einteilig durch einen Spritzgießprozess hergestellt werden.

Gemäß einer besonders bevorzugten Ausführungsform der Erfindung besteht der Behälter zumindest aus zwei Behälterteilen, wobei ein erster Behälterteil eine ebene oder gewölbte Rückwand und ein hiermit lösbar oder unlösbar verbundener zweiter Behälterteil eine ebene oder gewölbte Vorderwand umfasst. Wenn der Behälter zweiteilig ausgebildet ist, können die beiden Behälterteile auf besonders einfache Weise im Spritzgießverfahren hergestellt werden. Besonders bevorzugt sind die beiden Behälterteile mit einer Materialsbrücke miteinander verbunden.

Die lösbare Befestigung der beiden Behälterteile aneinander ermöglicht dabei eine leichte Auswechslung der Blisterverpackung, wenn nach dem Ablauf ihrer Wirkzeit eine ausreichende Beduftung der Umgebungsluft nicht mehr gegeben ist. Unter Rückwand ist dabei die Wand des Behälters zu verstehen, die in der Gebrauchsposition an der Stirnseite einer Papierrolle anliegt.

Besonders vorteilhaft ist es dabei, wenn der die Vorderwand aufweisende zweite Behälterteil eine radial innere und eine radial äußere Seitenwand umfasst, wobei die Seitenwände vorzugsweise über abgerundete Bereiche an die Vorderwand angeschlossen sind. Insbesondere kann dabei auch die Vorderseite eine Wölbung aufweisen, so dass der zweite Behälterteil insgesamt wulstartig ausgeformt ist. Dabei ist es weiterhin vorteilhaft, wenn auch der die Rückwand aufweisende erste Behälterteil einen stegartigen Randbereich aufweist, der für eine einfache Befestigung an den Seitenwänden des zweiten Behälterteils genutzt werden kann.

Wenn der Behälter eine Hülse der vorangehend beschriebenen Art aufweist, ist es weiterhin von Vorteil, wenn der erste Behälterteil, der die zumindest im wesentlichen eben ausgebildete Rückwand umfasst, mit der Hülse verbunden ist. Vorzugsweise entspricht dann der Innendurchmesser der Hülse in ihrem an die Rückwand angrenzenden Bereich dem Innendurchmesser der inneren Seitenwand des zweiten Behälterteils in ihrem an die Rückwand des ersten Behälterteils angrenzenden Bereich, so dass zwischen der Hülse und der inneren Seitenwand des Behälters ein stufenloser Übergang gegeben ist.

Eine besonders vorteilhafte und einfache Verbindung der beiden Behälterteile aneinander kann dadurch erreicht werden, dass sie formschlüssig und/oder kraftschlüssig, vorzugsweise mittels einer Rastverbindung oder Schnappverbindung aneinander befestigt sind. Auch können die beiden Behälterteile über eine oder mehrere Gewindeverbindungen oder mittels Haken und/oder Krampen aneinander befestigt werden.

Vorteilhafterweise können die Öffnungen in die Vorderwand und/oder in die radial außenliegende Seitenwand des ringförmigen Behälters eingebracht sein.

Hierdurch gelangen die Duftstoffe von Blisterverpackung besonders leicht und schnell an die Umgebungsluft, was zu einer besonders wirksamen Raumbeduftung führt. Die Vorderwand ist dabei die Wand des Behälters, die in der Gebrauchsposition von der Papierrolle abgewandt ist.

Die Blisterverpackung und der Aufnahmeraum sind bevorzugt in der Art konfiguriert, dass die mit der Duftstoff-permeablen Membran verschlossene Seite der Blisterverpackung an der von der Papierrolle abgewandten Seite im Aufnahmeraum positioniert ist.

Vorzugsweise sind die Öffnungen dabei als Schlitze, insbesondere wellenförmig ausgebildet. Sie können jedoch grundsätzlich beliebige Formgebungen aufweisen und insbesondere rund, oval oder tropfenförmig ausgeführt sein.

Gemäß einer weiteren bevorzugten Ausführungsform der Erfindung sind die Öffnungen oder die duftstoffpermeable Membran der Blisterverpackung mit entfernbaren Verschlussmitteln, insbesondere mit einer entfernbar aufgebrachten oder abziehbar aufgeklebten Folie verschlossen. Hierdurch wird eine unbeabsichtigte Duftabgabe vor Beginn der bestimmungsgemäßen Verwendung des Duftabgabesystems verhindert.

Vorzugsweise können auch perforierte Aufkleber als Verschlussmittel eingesetzt werden, wodurch die selektive Freigabe einzelner Bereiche von Öffnungen möglich ist, so dass beispielsweise zu Beginn der Wirkzeit des Duftabgabesystems zunächst nur einige wenige radial weiter innen liegende Öffnungen freigegeben werden und erst nach einer bestimmten Zeit und einem Nachlassen der Wirksamkeit weitere Öffnungen freigelegt werden, um eine vermehrte Duftabgabe und somit wieder eine zufrieden stellende Raumbeduftung zu erreichen.

Gemäß einer besonders bevorzugten Ausführungsform der Erfindung ist vorgesehen, dass ein verstellbarer Verschlussteil vorgesehen ist, durch welchen die Öffnungen des Behälters wahlweise vollständig oder teilweise geöffnet oder vollständig verschlossen werden können. Hierdurch können die Öffnungen auch nach dem Beginn der Benutzung des Duftabgabesystems wieder verschlossen werden, um seine Wirkzeit durch die Verhinderung einer weiteren Duftabgabe zu verlängern, wenn die Toilette beispielsweise urlaubsbedingt für einen bestimmten Zeitraum nicht benutzt wird.

Besonders vorteilhaft ist es dabei, wenn der Verschlussteil eine axial drehbar oder schwenkbar an dem ringförmigen Behälter angeordnete Kappe umfasst, die mit einer Mehrzahl von Öffnungen versehen ist, die wahlweise vollständig oder teilweise mit den Öffnungen des Behälters in Überdeckung gebracht werden können, wobei in zumindest einer Verschlussposition sämtliche Öffnungen des Behälters von den die Öffnungen der Kappe umgebenden Verschlussbereichen der Kappe verdeckt sind. Dabei können die Öffnungen in der Kappe auch so angeordnet sein, dass mit zunehmender Verdrehung der Kappe der Gesamt-Überdeckungsgrad mit den Öffnungen des Behälters kontinuierlich zu- bzw. abnimmt, um eine Dosierung der Duftabgabe vornehmen zu können. Bei einer zweiteiligen Behälterausführung ist die Kappe als dritter Bestandteil der erfindungsgemäßen Vorrichtung vorzugsweise an dem die Vorderwand enthaltenden Behälterteil angelenkt, insbesondere drehbar aufgeklipst.

Weiterhin schlägt die Erfindung vor, dass der Behälter aus einem durchsichtigen, transparenten oder semitransparenten Material gebildet ist. Auf diese Weise kann der Verbraucher den Füllgrad des Behälters und die optische Beschaffenheit sowie die Bewegung der Duftträgerpartikel erkennen. Insbesondere können hierbei auch verschiedene Farben von Duftträgerpartikeln erkannt und für die optische Gestaltung des Duftabgabesystems und/oder zu Unterscheidungszwecken bei verschiedenen Duftrichtungen genutzt werden. Alternativ hierzu kann der Behälter auch vollständig oder teilweise aus undurchsichtigem Material gebildet und galvanisiert, insbesondere verchromt sein, um einen besonders hochwertigen metallischen Eindruck zu erzeugen. Insbesondere kann der Behälter und die gegebenenfalls vorhandene Hülse aus einem Polymer oder einem Polymergemisch bestehen und vorteilhafterweise im Spritzgussverfahren hergestellt sein.

Als Parfümöle bzw. Duftstoffe können im Rahmen der vorliegenden Erfindung einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenyl-glycinat, Allylcyclohexylpropionat, Styrallylpropionat und Benzylsalicylat. Zu den Ethern zählen beispielsweise Benzylethylether, zu den Aldehyden z.B. die linearen Alkanale mit 8-18 C-Atomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller, Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl.

Die allgemeine Beschreibung der einsetzbaren Parfüme (siehe oben) stellt dabei allgemein die unterschiedlichen Substanzklassen von Riechstoffen dar. Um wahrnehmbar zu sein, muß ein Riechstoff flüchtig sein, wobei neben der Natur der funktionellen Gruppen und der Struktur der chemischen Verbindung auch die Molmasse eine wichtige Rolle spielt. So besitzen die meisten Riechstoffe Molmassen bis etwa 200 Dalton, während Molmassen von 300 Dalton und darüber eher eine Ausnahme darstellen. Auf Grund der unterschiedlichen Flüchtigkeit von Riechstoffen verändert sich der Geruch eines aus mehreren Riechstoffen zusammengesetzten Parfüms bzw. Duftstoffs während des Verdampfens, wobei man die Geruchseindrücke in "Kopfnote" (top note), "Herz- bzw. Mittelnote" (middle note bzw. body) sowie "Basisnote" (end note bzw. dry out) unterteilt. Da die Geruchswahrnehmung zu einem großen Teil auch auf der Geruchsintensität beruht, besteht die Kopfnote eines Parfüms bzw. Duftstoffs nicht allein aus leichtflüchtigen Verbindungen, während die Basisnote zum größten Teil aus weniger flüchtigen, d.h. haftfesten Riechstoffen besteht. Bei der Komposition von Parfüms können leichter flüchtige Riechstoffe beispielsweise an bestimmte Fixative gebunden werden, wodurch ihr zu schnelles Verdampfen verhindert wird. Bei der nachfolgenden Einteilung der Riechstoffe in "leichter flüchtige" bzw. "haftfeste" Riechstoffe ist also über den Geruchseindruck und darüber, ob der entsprechende Riechstoff als Kopf- oder Herznote wahrgenommen wird, nichts ausgesagt.

Durch eine geeignete Auswahl der genannten Duftstoffe bzw. Parfümöle kann auf diese Weise für erfindungsgemäße Mittel sowohl der Produktgeruch unmittelbar beim Öffnen des fabrikneuen Mittels als auch der Gebrauchsduft beeinflusst werden. Diese Dufteindrücke können selbstverständlich gleich sein, können sich aber auch unterscheiden. Für den letzteren Geruchseindruck ist die Verwendung haftfesterer Riechstoffe vorteilhaft, während zur Produktbeduftung auch leichterflüchtige Riechstoffe einsetzbar sind. Haftfeste Riechstoffe, die im Rahmen der vorliegenden Erfindung einsetzbar sind, sind beispielsweise die ätherischen Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaivabalsamöl, Korianderöl, Krauseminzeöl, Kümmelöl, Kuminöl, Lavendelöl, Lemongrasöl, Limetteöl, Mandarinenöl, Melissenöl, Moschuskömeröl, Myrrhenöl, Nelkenöl, Neroliöl, Niaouliöl, Olibanumöl, Orangenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronelöl, Zitronenöl sowie Zypressenöl.

Aber auch die höhersiedenden bzw. festen Riechstoffe natürlichen oder synthetischen Ursprungs können im Rahmen der vorliegenden Erfindung als haftfeste Riechstoffe bzw. Riechstoffgemische, also Duftstoffe, eingesetzt werden. Zu diesen Verbindungen zählen die nachfolgend genannten Verbindungen sowie Mischungen aus diesen: Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphthotmethylether, Nerol, Nitrobenzol, n-Nonylaldehyd, Nonylakohol, n-Octylaldehyd, p-Oxy-Acetophenon, Pentadekanolid, β-Phenylethylalkohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undelacton, Vanilin, Veratrumaldehyd, Zimtaldehyd, Zimtalkohol; Zimtsäure, Zimtsäureethylester, Zimtsäurebenzylester. Zu den leichter flüchtigen Riechstoffen zählen insbesondere die niedriger siedenden Riechstoffe natürlichen oder synthetischen Ursprung, die allein oder in Mischungen eingesetzt werden können. Beispiele für leichter flüchtige Riechstoffe sind Alkyisothiocyanate (Alkylsenföle), Butandion, Limonen, Linalool, Linaylacetat und -Propionat, Menthol, Menthon, Methyl-n-heptenon, Phellandren, Phenylacetaldehyd, Terpinylacetat, Zitral, Zitronellal.

Die in einer Blisterverpackung bevorratete Zubereitung kann außer den zur Beduftung und Desodorierung notwendigen Bestandteilen weitere Aktivsubstanzen enthalten. Von den Mitteln, welche ausschließlich der Beduftung dienen, lassen sich demnach weitere Produktgruppen unterscheiden, welche zusätzlich zu den vorgenannten erfindungsgemäßen Bestandteilen weitere bevorzugte Substanzen enthalten.

Eine dieser optional einsetzbaren bevorzugten Substanzen sind die Farbstoffe. Hierzu eignen sich generell sämtliche Farbstoffe, die dem Fachmann als geeignet zum Einfärben von Kunststoffen bzw. als löslich in Parfumölen bekannt sind. Es ist bevorzugt, den Farbstoff entsprechend des verwendeten Duftstoffs auszuwählen; beispielsweise weisen Partikel mit Zitronenduft vorzugsweise eine gelbe Farbe auf, während für Partikel mit Apfel- oder Kräuterduft eine grüne Farbe bevorzugt wird. Bevorzugte Farbstoffe besitzen eine hohe Lagerstabilität und Unempfindlichkeit gegenüber den übrigen Inhaltsstoffen der Mittel und gegen Licht.

Geeignete Farbstoffe und Farbstoffgemische sind unter verschiedenen Handelsnamen kommerziell erhältlich und werden unter anderem von den Firmen BASF AG, Ludwigshafen, Bayer AG, Leverkusen, Clariant GmbH, DyStar Textilfarben GmbH & Co. Deutschland KG, Les Colorants Wackherr SA und Ciba Specialty Chemicals angeboten. Zu den geeigneten fettlöslichen Farbstoffen und Farbstoffgemischen zählen beispielsweise Solvent Blue 35, Solvent Green 7, Solvent Orange 1 (Orange au Gras-W-2201), Sandoplast Blau 2B, Fettgelb 3G, Iragon^{®} Red SRE 122, Iragon^{®} Green SGR 3, Solvent Yellow 33 und Solvent Yellow 16, es können aber auch andere Farbstoffe enthalten sein.

In einer bevorzugten Ausführungsform besitzt der Farbstoff neben seiner ästhetischen Wirkung zusätzlich eine Indikatorfunktion. Hierdurch wird dem Konsumenten der aktuelle Verbrauchszustand des Duftes angezeigt, so dass er neben dem fehlenden Dufteindruck, der beispielsweise auch auf einem Gewöhnungseffekt seitens des Benutzers beruhen kann, ein weiteres zuverlässiges Anzeichen erhält, wann das Duftabgabesystem durch ein neues zu ersetzen ist.

Ferner können die Zubereitungen zur Bekämpfung von Mikroorganismen als weitere optional einsetzbare Substanz antimikrobielle Wirkstoffe enthalten. Hierbei unterscheidet man je nach antimikrobiellem Spektrum und Wirkungsmechanismus zwischen Bakteriostatika und Bakteriziden, Fungistatika und Fungiziden usw.. Wichtige Stoffe aus diesen Gruppen sind beispielsweise Benzalkoniumchloride, Alkylarlylsulfonate, Halogenphenole und Phenolmercuriacetat.

Um eine besonders gute Wirksamkeit der Desodorierung bzw. der Beduftung von Räumen zu erreichen, ist es besonders vorteilhaft, wenn zwei ringförmige Behälter vorgesehen sind, die an den beiden Stirnseiten einer Papierrolle, insbesondere einer Toilettenpapierrolle befestigt werden.

Im Sinne der Erfindung haben sich insbesondere zwei Riechstoffmischungen besonders bewährt. Zwar sind die positiven, erfindungsgemäßen Effekte prinzipiell mit jeglichen Riechstoffmischungen in sehr guter Weise erreichbar, jedoch konnten wir finden, dass die folgenden, bevorzugt einsetzbaren Riechstoffmischungen zu ganz besonders guten Resultaten in der Duftwahrnehmung bei der erfindungsgemäßen Anwendung führen.

Die bevorzugt einsetzbare Riechstoffmischung A zeichnet sich dadurch aus, dass sie
(a) mehr als 30 Gew.-%, vorzugsweise mehr 40 Gew.-%, insbesondere mehr als 50 Gew.-% an Riechstoffen mit einem clogP > 3,5, insbesondere ≥4 sowie einem Siedepunkt von < 250° C umfasst (wie insbesondere Decanal),
(b) mehr als 5 Gew.-%, vorzugsweise mehr als 10 Gew.-% an Riechstoffen mit einem clogP < 2 sowie einem Siedepunkt < 200°C (wie insbesondere Butylacetat),
(c) mehr als 10 Gew.-%, vorzugsweise mehr als 15 Gew.-%, insbesondere mehr als 20 Gew.-% an Riechstoffen mit einem clogP > 2,5 und < 3,5 sowie einem Siedepunkt von < 200° C (wie insbesondere Octanal).

Die Gew.-%-Angabe bezieht sich jeweils auf die Gesamtmenge der Riechstoffe, umfasst also nicht ggf. enthaltene Lösungsmittel wie z.B. Dipropylenglycol oder Isopar (Isoparaffine).

Die bevorzugt einsetzbare Riechstoffmischung B zeichnet sich dadurch aus, dass sie
(a) mehr als 20 Gew.%, vorzugsweise mehr als 30 Gew.-%, insbesondere mehr als 40 Gew.-% an Riechstoffen mit einem clogP > 3,5, insbesondere ≥ 4 sowie einem Siedepunkt von < 250° C umfasst (wie insbesondere 2-Methylundecanal, Isobornylacetat oder Nopylacetat)
(b) mehr als 10 Gew.-%, vorzugsweise mehr als 15 Gew.-%, insbesondere mehr als 20 Gew.-% an Riechstoffen mit einem clogP ≤ 3 sowie einem Siedepunkt von < 250° C (wie insbesondere Borneol oder z.B. auch Dihydromyrcenol mit einem clogP von 3,03) umfasst.

Die Gew.-%-Angabe bezieht sich wiederum auf die Gesamtmenge der Riechstoffe, umfasst also nicht ggf. enthaltene Lösungsmittel wie z.B. Dipropylenglycol oder Isopar (Isoparaffine).

Der ClogP-Wert ist dem Fachmann inzwischen auch aus der Patentliteratur gut bekannt. Er ist zurückzuführen auf den Octanol/Wasser-Verteilungskoeffizienten. Der logP-Wert zahlreicher Riechstoffe ist dokumentiert; zum Beispiel enthält die Pomona92-Datenbank, verfügbar von Daylight Chemical Information Systems, Inc., (Daylight CIS), Irvine, Kalifornien, zahlreiche logP-Werte, zusammen mit Zitaten zur Originalliteratur. Jedoch werden die logP-Werte am zweckmäßigsten durch das "CLOGP"-Programm, das auch von Daylight CIS verfügbar ist, berechnet. Dieses Programm führt auch die experimentellen logP-Werte auf, wenn sie in der Pomona92-Datenbank verfügbar sind. Der "errechnete logo" (das ist der ClogP-Wert) wird durch die Fragmentannäherung nach Harsch und Leo bestimmt (siehe A. Leo, in Comprehensive Medicinal Chemistry, Bd. 4, C. Harsch, P. G. Sammens, J. B. Taylor und C. A. Ransden, Hrsg., S. 295, Pergamon Press, 1990, hierin durch den Bezug eingeschlossen). Die Fragmentannäherung basiert auf der chemischen Struktur jedes der Duftstoffbestandteile und berücksichtigt die Zahlen und Typen von Atomen, die Atombindungsfähigkeit und die chemische Bindung. Die ClogP-Werte, welches die zuverlässigsten und am verbreitesten verwendeten Schätzwerte für diese physikochemische Eigenschaft sind, werden im Rahmen dieser Erfindung vorzugsweise an Stelle der experimentellen logP-Werte bei der Auswahl der Duftstoffbestandteile verwendet, die in der vorliegenden Erfindung mit Vorteil einsetzbar sind. Liegen experimentelle logP vor, so ist es möglich, den experimentellen logP-Wert an Stelle des ClogP-Wert zu verwenden.

Vorzugsweise wird das erfindungsgemäße Duftabgabesystemen zur Beduftung von Badezimmern oder Toilettenräumen verwendet. Es kann jedoch auch an Küchenpapierrollen bzw. Haushaltspapierrollen zur Beduftung eines Küchenraums oder eines sonstigen Arbeitsraums dienen, in dem eine solche Papierrolle eingesetzt wird.

Weitere Vorteile und Merkmale der Erfindung ergeben sich aus der nachfolgenden Beschreibung und den in den Zeichnungen dargestellten Ausführungsbeispielen. Es zeigen:
- Fig. 1: Ringförmiger Behälter in geöffneter Stellung in Seitenansicht
- Fig. 2: Ringförmiger Behälter in geöffneter Stellung in der Aufsicht
- Fig. 3: Ringförmiger Behälter in geöffneter Stellung in zwei perspektivischen Ansichten
- Fig. 4: Blisterverpackung mit Zubereitung in horizontaler und vertikaler Stellung
- Fig. 5: Blisterverpackung in partieller Querschnittsansicht

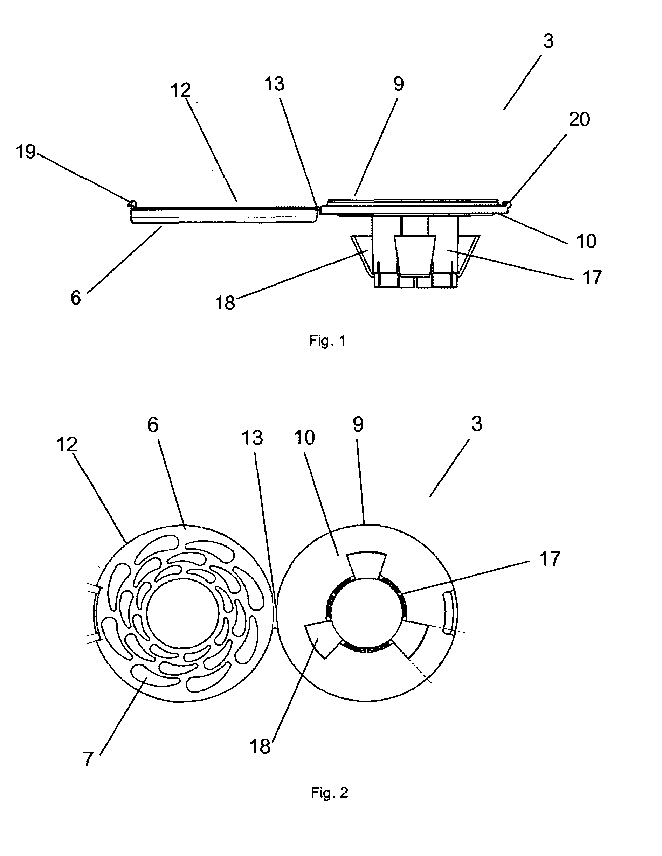

Fig.1 zeigt den ringförmigen Behälter 3 in geöffneter Stellung. Der Behälter 3 ist zweiteilig ausgeführt, wobei der der erste Behälterteil 9 eine Rückwand 10 umfasst von der aus sich eine koaxial hervorstehende Hülse 17 vertikal vom Behälterteil 9 weg erstreckt. Über die elastisch verformbare Materialbrücke 13 ist an dem ersten Behälterteil 9 ein zweiter Behälterteil 12 angeformt, der die Vorderwand 6 des Behälters 6 umfasst. Durch Schwenken des zweiten Behälterteils 12 um die Materialbrücke 13 lässt sich der Behälter 3 in seine Verschlussstellung bringen, wobei am ersten Behälterteil 9 und am zweiten Behälterteil 12 jeweils Verrastungsmittel 19,20 vorgesehen sind, die den ersten und zweiten Behälterteil 9,12 in der Verschlussstellung lösbar fixieren.

In der Verschlussstellung des ersten und zweiten Behälterteils 9,12 ist im Behälter ein ringförmiger Aufnahmeraum 4 (nicht in Fig. 1 gezeigt) ausgebildet, in den eine korrespondierende, mit Duftstoff befüllte Blisterverpackung 11 eingesetzt werden kann. Dies wird an nachfolgender Stelle noch näher erläutert.

An der koaxial vorstehenden Hülse 17 sind Befestigungsmittel 18 in Form von Federlaschen angeordnet, die eine rotatorische Befestigung des Behälters 3 an einer Papierrolle 2 und/oder einem Kernrohr einer Papierrolle erlauben. Rotatorische Befestigung bedeutet, dass die Befestigung derart konfiguriert ist, dass bei einer Drehung der Papierrolle 2 - beispielsweise beim Abrollen von Papier - der Behälter 3 mit gedreht wird. Der Fachmann wird hierzu die Federkräfte der Federlaschen 18 entsprechend anpassen.

Die Hülse 17 und die Befestigungselemente 18 sind derart ausgeführt, dass sie axial in eine Papierrolle 2 und/oder in ein Kemrohr einer Papierrolle 2 einführbar sind.

Fig. 2 zeigt den aus Fig. 1 bekannten Behälter 3 in einer Aufsicht. Man erkennt, dass die Vorderwand 6 des Behälters 3 mit Öffnungen 7 versehen ist, die eine leichte Abgabe von Duftstoff in die Umgebung erlauben. Im verschlossenen und mit einer Blisterverpackung 11 befüllten Zustand des Behälters 3 ist der Behälter 3 und die Blisterverpackung 11 in der Art ausgebildet, dass die mit einer Duftstoff-permeablen Membran 15 ersehene Seite der Blisterverpackung 11 an der Vorderwand 6 des Behälters 3 positioniert ist.

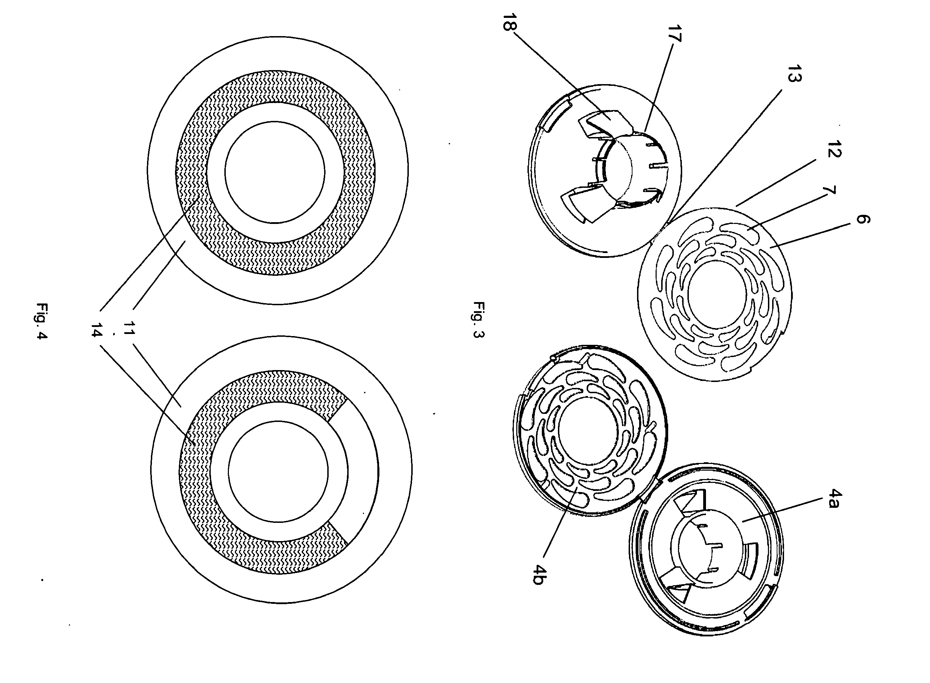

Das erste und zweite Gehäuseteil 9,12 bilden im geschlossenen Zustand einen Aufnahmeraum 4 für eine korrespondierende Blisterverpackung 11 aus. Dies ist gut anhand von Fig. 3 zu erkennen, in der der geöffnete Behälter 3 in einer perspektivischen Ansicht gezeigt ist. Man erkennt an der rechten Abbildung, dass das erste und zweite Gehäuseteil 9,12 jeweils einen schalenartigen Raum 4a,4b definieren. Durch Verschluss des Behälters 3 durch Schwenken der Gehäuseteile 9,12 in die Verschlussstellung, wird der ringförmige Aufnahmeraum 4 für die mit Duftstoff befüllte Blisterverpackung 11 gebildet.

Die Blisterverpackung 11 und der Behälter 3 sind dabei so ausgeformt, dass die Blisterverpackung 11 in der Verschlussstellung des Behälters 3 bei einer Drehbewegung des Behälters 3 um seine durch die Hülse 17 definierten Rotationsachse mit gedreht wird. Somit wird sichergestellt, dass die viskose Zubereitung beim Drehen der Papierrolle und somit des Behälters 3 die Duftstoff-permeable Membran 15 möglichst über ihre gesamte benetzbare Fläche benetzt.

Fig.4 zeigt eine Blisterverpackung 11 mit einer Duftstoff enthaltenen Zubereitung 14. Die linke Abbildung zeigt die Blisterverpackung 11 in einer liegenden, horizontalen Lage, die rechte Abbildung zeigt die Blisterverpackung 11 in einer stehenden, vertikalen Lage, wie sie üblicherweise vorherrscht, wenn sich die Blisterverpackung 11 in einem Behälter 3 befindet, der bestimmungsgemäß an einer Papierrolle befestigt ist.

Man erkennt an der rechten Abbildung, dass die Blisterverpackung 11 nicht vollständig mit Zubereitung 14 befüllt ist. Der Füllgrad der Blisterverpackung 11 mit Zubereitung 14 beträgt weniger als 95% des maximalen Füllvolumens der Blisterverpackung 11.

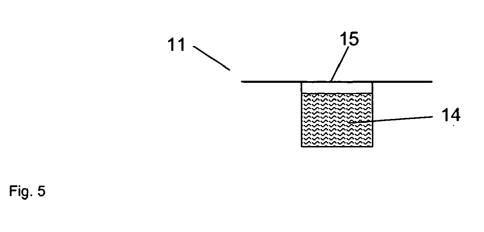

Fig. 5 zeigt einen Querschnitt durch die aus Fig. 4 bekannte Blisterverpackung 11 entlang der Schnittebene A-A. Die Blisterverpackung 11 weist einen napfartigen Aufnahmeraum für die Zubereitung 14 auf, der durch die Duftstoff-permeable Membran 15 verschlossen ist, so dass nur Duftstoff, jedoch keine Flüssigkeit oder Gel aus der Blisterverpackung 11 austreten kann.

## Patentansprüche

1. Duftabgabesystem (1) zur Desodorierung und/oder zur Beduftung von Räumen an drehbar gelagerten Papierrollen (2), umfassend einen mit einer Mehrzahl von Öffnungen (7, 8a) versehenen ringförmigen Behälter (3), der einen ringförmigen Aufnahmeraum (4) umschließt,
• wobei die Öffnungen (7) eine Emittierung der Duftstoffe vom Aufnahmeraum (4) nach außen ermöglichen, und
• dass der Behälter (3) eine koaxial vorstehende Hülse (17) aufweist, die zur vorzugsweise konzentrischen Positionierung des Behälters (3) an einer Stimseite einer Papierrolle (2) axial in eine Papierrolle (2) oder in ein Kernrohr einer Papierrolle (2) einsteckbar ist, und
• dass der Behälter (3) kraftschlüssig und/oder formschlüssig wirkende Befestigungsmittel (18, 20) zur rotatorischen Befestigung des Behälters (3) an einer Papierrolle (2) und/oder an einem Kemrohr einer Papierrolle (2) aufweist,
• wobei der Behälter (3) zumindest zweiteilig ausgeführt ist, wobei ein erster Behälterteil (9) eine ebene oder gewölbte Rückwand (10) und ein hiermit lösbar verbundener zweiter Behälterteil (12) eine ebene oder gewölbte Vorderwand (6) umfasst,
• wobei der erste Behälterteil (9) und der zweite Behälterteil (12) über eine schamierartige Materialbrücke (13) miteinander verbunden sind,
**dadurch gekennzeichnet, dass** in den ringförmigen Aufnahmeraum (4) eine korrespondierende, ringförmige mit einer Duftstoff-permeablen Membran (15) verschlossene und mit einer fließfähigen, wenigstens einen Duftstoff enthaltenden Zubereitung (14) befüllte Blisterverpackung (11) eingesetzt ist, so dass die Blisterverpackung (11) in der Verschlussstellung des ringförmigen Aufnahmeraums (4) bei einer Drehbewegung des Behälters (3) mitgedreht wird und die kinematische Viskosität der in der Blisterverpackung (11) bevorrateten Zubereitung (14) bei 40°C zwischen 1,4 und 150 mm²/s, bevorzugt 1,4 und 44 **mm²/s** liegt, wobei das Volumen der Blisterverpackung (11) mit weniger als 95% an Zubereitung (14) befüllt ist.

2. Duftabgabesystem nach Anspruch 1, **dadurch gekennzeichnet, dass** der Behälter (3) eine koaxial vorstehende Hülse (17) aufweist, die von einer Stirnseite einer Papierrolle axial in die Papierrolle oder in das Kernrohr einer Papierrolle einsteckbar ist.

3. Duftabgabesystem nach Anspruch 2, **dadurch gekennzeichnet, dass** die Hülse (17) konusförmig ausgebildet ist wobei ihr freies Ende mit einem kleineren Außendurchmesser versehen ist.

## Claims

1. A scent release system (1) for deodorising and/or fragrancing rooms on rotatably mounted rolls of paper (2) comprising an annular container (3) provided with a plurality of orifices (7, 8a), which container encloses an annular accommodation space (4),
• the orifices (7) enabling emission of scents from the accommodation space (4) to the outside, and
• the container (3) comprising a coaxially projecting sleeve (17) which, for preferably concentric positioning of the container (3) on an end face of a roll of paper (2), is insertible axially into a roll of paper (2) or into a core tube of a roll of paper (2), and
• the container (3) comprising fastening means (18, 20) with a frictional and/or interlocking action for securing the container (3) for rotation to a roll of paper (2) and/or to a core tube of a roll of paper (2),
• the container (3) being constructed in at least two parts, a first container part (9) comprising a planar or curved rear wall (10) and a second container part (12) detachably connected thereto comprising a planar or curved front wall (6),
• the first container part (9) and the second container part (12) being connected to one another via a hinge-like material bridge (13),
**characterised in that** a corresponding, annular blister pack (11) closed with a scent-permeable membrane (15) and filled with a flowable preparation (14) containing at least one scent is inserted into the annular accommodation space (4), such that, in the closure position of the annular accommodation space (4), the blister pack (11) is co-rotated on rotational motion of the container (3) and the kinematic viscosity of the preparation (14) stored in the blister pack (11) is between 1.4 and 150 mm²/s, preferably 1.4 and 44 mm²/s, at 40°C, the volume of the blister pack (11) being less than 95% filled with preparation (14).

2. A scent release system according to claim 1, **characterised in that** the container (3) comprises a coaxially projecting sleeve (17) which is axially insertible from an end face of a roll of paper into the roll of paper or into the core tube of a roll of paper.

3. A scent release system according to claim 2, **characterised in that** the sleeve (17) is of conical construction, the free end thereof being provided with a smaller external diameter.

## Revendications

1. Système de libération (1) de parfum, pour désodoriser et/ou parfumer des espaces sur des rouleaux de papier (2) logés de manière à pouvoir tourner, comprenant un récipient (3) annulaire pourvu d'une pluralité d'ouvertures (7, 8a), qui enferme un espace de logement (4) annulaire,
- les ouvertures (7) permettant une émission des parfums de l'espace de logement (4) vers l'extérieur, et
- en ce que le récipient (3) présente un manchon (17) coaxialement proéminent, qui peut être inséré sur une face frontale d'un rouleau de papier, axialement dans un rouleau de papier (2) ou dans un tube formant le noyau d'un rouleau de papier (2) pour le positionnement de préférence concentrique du récipient (3), et
- en ce que le récipient (3) présente des moyens de fixation (18, 20) agissant par assemblage par force et/ou par forme pour la fixation rotatoire du récipient (3) sur un rouleau de papier (2) et/ou sur un tube formant le noyau d'un rouleau de papier (2),
- le récipient (3) étant conçu en au moins deux parties, une première partie (9) de récipient comprenant une paroi arrière (10) plane ou incurvée et une deuxième partie (12) de récipient assemblée de manière amovible avec celle-ci présentant une paroi avant (6) plane ou incurvée,
- la première partie (9) de récipient et la deuxième partie (12) de récipient étant reliées l'une à l'autre via un pont en matière (13), de type charnière,
**caractérisé en ce qu'**un emballage blister (11) correspondant, annulaire, fermé par une membrane (15) perméable au parfum et rempli d'une préparation (14) pouvant s'écouler, contenant au moins un parfum, est placé dans l'espace de logement (4) annulaire, de manière telle que l'emballage blister (11) est entraîné en rotation, dans la position verrouillée de l'espace de logement (4) annulaire, lors d'un mouvement de rotation du récipient (3) et la viscosité cinématique de la préparation (14) approvisionnée dans l'emballage blister (11), à 40°C, se situe entre 1,4 et 150 mm²/s, de préférence entre 1,4 et 44 mm²/s, le volume de l'emballage blister (11) étant rempli à moins de 95% de préparation (14).

2. Système de libération de parfum selon la revendication 1, **caractérisé en ce que** le récipient (3) présente un manchon (17) coaxialement proéminent, qui peut être inséré à partir d'une face frontale d'un rouleau de papier axialement dans le rouleau de papier ou dans le tube formant le noyau d'un rouleau de papier.

3. Système de libération de parfum selon la revendication 2, **caractérisé en ce que** le manchon (17) est conçu en forme de cône, son extrémité libre étant pourvue d'un diamètre externe plus petit.
